# EUROPÄISCHE PATENTSCHRIFT

(11) **EP 3 773 802 B1**
(45) Veröffentlichungstag und Bekanntmachung des Hinweises auf die Patenterteilung: **03.11.2021**
(21) Anmeldenummer: 19714178.1
(22) Anmeldetag: 26.03.2019
(51) Int. Cl.: A61M 5/20, A61M 5/30, A61M 5/31

(54) **VORRICHTUNG ZUM APPLIZIEREN EINES FLUIDS**
DEVICE FOR APPLYING A FLUID
DISPOSITIF D'APPLICATION D'UN FLUIDE

(30) Priorität: 26.03.2018 DE 102018107100
(43) Veröffentlichungstag der Anmeldung: 17.02.2021
(73) Patentinhaber: Intervet International B.V., 5831 AN Boxmeer (NL)
(72) Erfinder: ALTERMANN, Frank, 78532 Tuttlingen (DE); KAMENZIN, Dominic, 78253 Eigeltingen-Reute (DE); MATTES, Manuel, 78549 Spaichingen (DE); SAUTER, Robin, 78532 Tuttlingen (DE); SCHMIDT, Thomas, 78532 Tuttlingen (DE); SEEH, Daniel, 78194 Immendingen (DE)
(74) Vertreter: Intervet International B.V.
(86) Internationale Anmeldenummer: PCT/EP2019/057533
(87) Internationale Veröffentlichungsnummer: WO 2019/185602

(56) Entgegenhaltungen:
- WO-A1-2007/140610
- US-A- 3 057 349
- US-A- 3 353 537
- US-A- 3 973 697
- US-A- 4 103 684
- US-A1- 2011 224 613

## Beschreibung

Die vorliegende Erfindung betrifft eine Vorrichtung zum Applizieren eines Fluids, die beispielsweise als nadellose Selbstfüllerspritze ausgebildet sein kann, mit der Tieren ein flüssiges Arzneimittel verabreicht werden kann.

Solche Vorrichtungen zum Applizieren eines Fluids sollen möglichst leicht, langlebig und mit geringen Wartungskosten und -aufwand verbunden sein.

Aufgabe der Erfindung ist es, eine Vorrichtung zum Applizieren eines Fluids bereitzustellen, die mindestens eine der genannten Eigenschaften verwirklicht.

Die Erfindung ist im Anspruch 1 definiert. Vorteilhafte Ausgestaltungen sind in den abhängigen Ansprüchen angegeben.

Die erfindungsgemäße Vorrichtung zum Applizieren eines Fluids umfasst einen Zylinder, der ein offenes Abgabeende aufweist, einen im Zylinder zwischen einer vorderen und einer hinteren Endposition verschiebbaren Kolben, der mit einer Kolbenstange verbunden ist, die entlang einer ersten Richtung über ein dem offenen Abgabeende entgegengesetztes hinteres Ende des Zylinders heraussteht und in einem Aufnahmeblock geführt ist, ein das offene Abgabeende verschließendes Rückschlagventil (bzw. Ansaugventil) sowie eine mit der Kolbenstange verbundene Spannvorrichtung, die im Aufnahmeblock angeordnet ist. Die Spannvorrichtung kann die Kolbenstange in einem Spannvorgang, wenn der Kolben in seiner vorderen Endposition steht, entlang der ersten Richtung bewegen, bis der Kolben in seiner hinteren Endposition steht, um den Zylinder dadurch mit dem zu applizierenden Fluid zu füllen und die Kolbenstange zum offenen Abgabeende hin vorzuspannen. Ferner kann die Spannvorrichtung, wenn der Kolben in seiner hinteren Endposition steht, die Kolbenstange in einem Abgabevorgang freigeben, so dass der Kolben aufgrund der anliegenden Vorspannung entgegen der ersten Richtung bis zu seiner vorderen Endposition bewegt und dabei das Fluid im Zylinder über das Rückschlagventil zum Applizieren abgegeben wird. Erfindungsgemäß ist der Zylinder zusammen mit dem Rückschlagventil als austauschbare Frontgruppe, die auch als austauschbare Gruppe bezeichnet werden kann, ausgebildet, die mit dem Aufnahmeblock lösbar verbunden ist.

Damit kann leicht die gesamte Frontgruppe, die dem größten Verschleiß beim Betrieb der Vorrichtung zum Applizieren eines Fluids (bevorzugt einer Flüssigkeit) unterliegt, in eine neue (bevorzugt baugleiche) Frontgruppe getauscht werden, die dann mit dem Aufnahmeblock verbunden wird. Damit wird die Haltbarkeit der gesamten Vorrichtung zum Applizieren eines Fluids deutlich erhöht.

Die austauschbare Gruppe kann am vorderen Ende der erfindungsgemäßen Vorrichtung angeordnet sein. Insbesondere kann z.B. ein Teil der austauschbaren Gruppe bei bestimmungsgemäßen Gebrauch der erfindungsgemäßen Vorrichtung in Kontakt mit dem Tier stehen, dem das Fluid appliziert werden soll. Insofern steht zumindest dieser Teil der austauschbaren Gruppe von der restlichen erfindungsgemäßen Vorrichtung vor. Die austauschbare Gruppe kann einen Teil aufweisen, der das distale Ende der erfindungsgemäßen Vorrichtung bildet, so dass z.B. aus diesem Grund die austauschbare Gruppe auch als austauschbare Frontgruppe bezeichnet werden kann.

Unter einer austauschbaren Frontgruppe wird hier insbesondere verstanden, dass die Frontgruppe als Ganzes vom Aufnahmeblock getrennt und durch eine baugleiche Frontgruppe ersetzt werden kann, die zum Austausch mit dem Aufnahmeblock verbunden wird. Es ist jedoch auch möglich, dass die vom Aufnahmeblock getrennte Frontgruppe gewartet wird (indem z.B. Verschleißteile wie Dichtungen ausgetauscht werden) und danach wieder mit dem Aufnahmeblock verbunden wird.

Da die Frontgruppe komplett vom Aufnahmeblock getrennt wird und dann gewartet oder ausgetauscht wird, können unerwünschte Kontaminationen sicher vermieden werden. Dies ist deutlich schwieriger und mit höheren Kosten verbunden, wenn z.B. lediglich die erst verschleißenden O-Ring-Dichtungen in der Frontgruppe einzeln ausgetauscht werden, wenn die Frontgruppe, wie bisher üblich, so verbaut ist, dass sie nicht zerstörungsfrei von der restlichen Applizierungsvorrichtung getrennt werden kann.

Die lösbare Verbindung zwischen der Frontgruppe und dem Aufnahmeblock kann insbesondere eine Schraubverbindung sein. Es ist jedoch auch jede andere Art der lösbaren Verbindung, wie z.B. eine Bajonett-Verbindung, möglich.

Die erfindungsgemäße Applizierungsvorrichtung weist eine Düse zum nadellosen Applizieren des

Fluids auf, die über das Rückschlagventil mit dem offenen Abgabeende des Zylinders verbunden ist und die Bestandteil der Frontgruppe ist. Damit ist es möglich, beim Austausch der Frontgruppe auch gleich die Düse mit auszutauschen.

Alternativ kann die Vorrichtung eine Nadel oder Kanüle aufweisen, die über das Rückschlagventil mit dem offenen Abgabeende des Zylinders verbunden ist und die Bestandteil der Frontgruppe ist. Die Nadel und die Kanüle können ihrerseits austauschbar sein.

Die erfindungsgemäße Applizierungsvorrichtung kann genau einen Zylinder mit genau einer Kolbenstange und genau einer Frontgruppe aufweisen. Es ist jedoch auch möglich, dass die Applizierungsvorrichtung zwei oder mehr Zylinder mit zwei oder mehreren Kolbenstangen sowie zwei oder mehrere Frontgruppen aufweist, die alle gleich ausgebildet sind, so dass zwei oder mehr gleiche oder unterschiedliche Fluide gleichzeitig beaufschlagt werden können. Die einzelnen Zylinder können gleiches oder unterschiedliches Volumen aufweisen.

Die erfindungsgemäße Applizierungsvorrichtung ist insbesondere als Selbstfüller-Applizierungsvorrichtung ausgebildet, die den Spannvorgang dazu nutzt, dass der Zylinder mit dem zu applizierenden Fluid (z.B. eine zu applizierende Flüssigkeit) gefüllt wird.

Dies kann z.B. so realisiert sein, dass während des gesamten Spannvorgangs schon ein Auffüllen des Zylinders stattfindet. Alternativ kann die Applizierungsvorrichtung so ausgebildet sein, dass während des Spannvorgangs ein Unterdruck in dem Zylinder aufgebaut wird, der dann, wenn der Kolben in seiner hinteren Endposition steht, dazu benutzt wird, dass das Fluid aufgrund des Unterdrucks in den Zylinder gesaugt wird. Dazu kann beispielsweise der Kolben in seinem distalen Ende eine sich in Längsrichtung der Kolbenstange erstreckende Sackbohrung aufweisen, von der ein oder mehrere radiale Bohrungen abzweigen, die in der hinteren Endposition des Kolbens eine Fluidverbindung zu einem Reservoir des zu applizierenden Fluids herstellen.

Die erfindungsgemäße Applizierungsvorrichtung kann den Motor aufweisen, der zum Durchführen des Spannvorganges genutzt wird. Man kann auch sagen, dass über den Motor die Energie bereitgestellt wird, die zum Aufbau der Vorspannung der Kolbenstange notwendig ist. Der Motor kann insbesondere am Aufnahmeblock gelagert sein.

Zum Betrieb des Motors sowie etwaiger weiterer Verbraucher kann als Energiequelle z.B. eine Batterie und/oder ein Akkumulator, der wiederaufladbar ist, vorgesehen sein. Die Energiequelle kann z.B. im Fuß oder als Fuß der Applizierungsvorrichtung ausgebildet sein. Ferner kann die Energiequelle austauschbar oder fest eingebaut sein.

Die Spannvorrichtung kann eine Feder aufweisen, die die Kolbenstange zum offenen Abgabeende hin vorspannt, wenn der Kolben in der hinteren Endposition steht.

Die erfindungsgemäße Applizierungsvorrichtung kann ferner eine mittels des Motors drehbare Rampe mit einer sich entlang einer Schraubenlinie erstreckenden Rampenbahn aufweisen. Die Rampenbahn kann von einem ersten Plateau oder Niveau entlang eines Steigungsbereiches zu einem zweiten Plateau oder zweiten Niveau ansteigen und vom zweiten Plateau über eine Sprungflanke zum ersten Plateau abfallen. Die Rampenbahn weist somit eine einzige Windung auf und kann als wendelförmig mit einer Stufe bezeichnet werden.

Die Spannvorrichtung kann ferner eine die Rampenbahn kontaktierende Walze, die in einen Mitnehmer, der mit dem aus dem Zylinder herausstehenden Ende der Kolbenstange verbunden ist, drehbar gelagert ist, aufweisen, so dass bei Drehung der Rampe die Rampenbahn unter der sich dadurch drehenden Walze durchläuft. Die Walze ist bevorzugt so gelagert, dass ihre Drehachse senkrecht zur ersten Richtung (bzw. senkrecht zur Längsachse der Kolbenstange) ist.

Für den Spannvorgang kann die Rampenbahn ausgehende von einem Kontakt der Walze mit dem ersten Plateau so gedreht werden, dass die Walze auf dem Steigungsbereich bis zum zweiten Plateau läuft und dadurch der Kolben in seine hintere Endposition bewegt wird. Für den Abgabevorgang kann die Rampenbahn ausgehend von einem Kontakt der Walze mit dem zweiten Plateau so lange gedreht werden, bis die Walze über die Sprungflanke auf das erste Plateau trifft und dadurch der Kolben in seine vordere Endposition bewegt wird.

Der Abstand des zweiten Plateaus vom ersten Plateau entlang der Drehachse der Rampe entspricht bevorzugt dem Abstand von der vorderen zur hinteren Endposition des Kolbens entlang der ersten Richtung und somit dem Kolbenhub.

Mittels der Rampe und der Walze wird somit die Drehbewegung des Motors in eine translatorische Bewegung der Kolbenstange entlang ihrer Längsachse umgesetzt. Es kann damit die Applizierungsvorrichtung mittels des Motors gespannt werden, so dass ein Benutzer lediglich ein Auslöseelement, wie z.B. eine Taste, ein Schalter, eine Schaltwippe oder einen Knopf, betätigen muss, um den Abgabevorgang freizugeben und damit das Applizieren des Fluids zu bewirken. Damit können z.B. viele Tiere schnell nacheinander mit einem Medikament geimpft werden.

Der Steigungsbereich der Rampenbahn kann einen an das erste Plateau anschließenden ersten Abschnitt und einen daran anschließenden zweiten Abschnitt aufweisen, wobei die Steigung des zweiten Abschnitts größer ist als die Steigung des ersten Abschnitts.

Damit wird vorteilhaft erreicht, dass zu Beginn des Spannvorgangs eine geringere Steigung der Rampenbahn vorliegt wodurch der Motor weniger oder Drehmoment aufbringen muss. Dies ist beim Anlaufen des Motors von Vorteil, da beim Anlaufen mehr Strom gezogen wird. Sobald der erste Abschnitt in den zweiten Abschnitt übergeht, ist diese Anlaufproblematik überwunden, so dass hier eine größere Steigung leicht realisiert werden kann. Dies erhöht die Haltbarkeit des Motors.

Der Steigungsbereich der Rampenbahn kann so ausgebildet sein, dass beide Abschnitte bezogen auf den Drehwinkel der Schraube linear sind. Es ist jedoch auch möglich, dass der erste Abschnitt und/oder der zweite Abschnitte einen nicht-linearen Verlauf bezogen auf den Drehwinkel aufweisen. In diesem Fall ist die Steigung des jeweiligen Abschnitts bevorzugt die mittlere Steigung des jeweiligen Abschnitts. Der nicht-lineare Verlauf des jeweiligen Abschnitts ist bevorzugt ein Verlauf, bei dem die lokale Steigung mit zunehmenden Drehwinkel zunimmt. Der nicht-lineare Verlauf des jeweiligen Abschnitts kann bevorzugt ein konkaver Krümmungsverlauf sein.

Insbesondere kann der Drehwinkelbereich (bzw. die Drehwinkellänge) des ersten Abschnitts kleiner sein als der Drehwinkelbereich (bzw. die Drehwinkellänge) des zweiten Abschnitts. Bevorzugt beträgt das Verhältnis des Drehwinkelbereichs des ersten Abschnitts zum Drehwinkelbereich des zweiten Abschnitts nicht mehr als 4/6 und nicht weniger als 1/9.

Bei der erfindungsgemäßen Applizierungsvorrichtung kann die Walze einen Auflagebereich, der auf dem Steigungsbereich der Rampenbahn aufliegt, und mindestens einen sich daran seitlich anschließenden Seitenbereich, der einen geringeren Außendurchmesser als der des Auflagebereiches aufweist und der nicht auf dem Steigungsbereich der Rampenbahn aufliegt, aufweisen. Beim Abgabevorgang kann sowohl der Auflagebereich als auch der Seitenbereich in Kontakt mit einer das zweite Plateau mit der Sprungflanke verbindenden Kante der Rampenbahn kommen. Dies führt zu dem Vorteil, dass ein relativ geringer Roll- oder Reibwiderstand zwischen der Walze und der Rampenbahn im Bereich des Steigungsbereiches vorliegt. Beim Übergang über die Kante für den Abgabevorgang liegt dann sowohl der Aufnahmebereich als auch der Seitenbereich auf der Kante auf, so dass hier die Auflagefläche vergrößert ist, so dass ein kleinerer Druck vorliegt. Dies ist von Vorteil, da beim Übergang der Walze über die Kante die höchste Kraft auf die Walze einwirkt, und somit unerwünschte Druckspitzen vermindert werden können. Damit wird die Haltbarkeit der Walze erhöht.

Insbesondere kann die Walze auf beiden Seiten des Auflagebereichs je einen seitlich anschließenden Seitenbereich mit geringerem Außendurchmesser als der des Auflagebereiches aufweisen. Dies führt zu einer weiteren Verringerung des Drucks auf die Walze beim Übergang über die Kante.

Die Walze kann so ausgebildet sein, dass der Außendurchmesser des Auflagebereichs konstant ist. Der Außendurchmesser des jeweiligen Seitenbereichs kann in einer Richtung zur Seite der Walze hin (bzw. in einer Richtung weg vom Auflagebereich oder mit größer werdendem Abstand vom Auflagebereich) abnehmen.

Die Walze kann als Kunststoff-Walze ausgebildet sein. Die Rampe kann aus Metall bestehen.

Bei der erfindungsgemäßen Applizierungsvorrichtung kann der Aufnahmeblock als einstückiger Aufnahmeblock ausgebildet sein, der durch ein additives Herstellungsverfahren hergestellt ist.

Ein solches additives Herstellungsverfahren kann auch als 3D-Drucken bezeichnet werden und z.B. ein Lasersinterverfahren sein.

Dies führt dazu, dass der Aufnahmeblock mit relativ geringem Gewicht und hoher Steifigkeit und Festigkeit hergestellt werden kann. Damit wird das Gesamtgewicht der Applizierungsvorrichtung möglichst gering gehalten, wodurch ein angenehmes dauerhaftes Bedienen für einen Benutzer möglich ist.

Ferner führt die einstückige Ausbildung des Aufnahmeblocks mittels des additiven Herstellungsverfahrens in vorteilhafter Weise dazu, dass der Aufnahmeblock äußerst kompakt gestaltet werden kann, was mit herkömmlichen spanabhebenden Herstellungsverfahren nicht möglich ist.

Als Material für den Aufnahmeblock wird bevorzugt ein Metall (oder eine Metall-Legierung) und insbesondere Titan verwendet, so dass der Aufnahmeblock aus Metall (oder einer Metall-Legierung) und insbesondere aus Titan besteht. Ferner kann als Material für den Aufnahmeblock Aluminium, Stahl (z.B. Maraging-Stahl), Edelstahl, Titan, eine Nickel-Legierung und/oder eine Kobalt-Chrom-Legierung verwendet werden. Weitere mögliche Materialien sind AlSiMg-Legierungen, CoCrMo-Legierungen sowie Nickel-Chrom-Legierungen. Ferner können Materialien verwendet werden, die schweißbar sind. Alle diese Materialien können in einer Form (z.B. als Pulver) vorliegen, um aus ihnen mittels eines additiven Herstellungsverfahrens (und insbesondere mittels des Lasersinterns) den Aufnahmeblock herstellen zu können.

Der Aufnahmeblock kann ein Motorlager, einen Führungszylinder für die Kolbenstange, mindestens eine Aufnahme für eine Steuerungsplatine, eine Aufnahme für einen Fluidanschluss für einen Fluidbehälter und/oder mindestens eine Gehäusefixierstelle aufweist, die einstückig mit dem Aufnahmeblock ausgebildet ist/sind.

Der Kolben kann einstückig mit der Kolbenstange ausgebildet sein. In diesem Fall bildet das vordere Ende der Kolbenstange den Kolben. Es ist jedoch auch möglich, dass der Kolbe ein separates Element ist, dass mit der Kolbenstange verbunden ist.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung anhand von Ausführungsbeispielen unter Bezugnahme auf die beigefügten Zeichnungen, die ebenfalls erfindungswesentliche Merkmale offenbaren, noch näher erläutert. Diese Ausführungsbeispiele dienen lediglich der Veranschaulichung und sind nicht als einschränkend auszulegen. Beispielsweise ist eine Beschreibung eines Ausführungsbeispiels mit einer Vielzahl von Elementen oder Komponenten nicht dahingehend auszulegen, dass alle diese Elemente oder Komponenten zur Implementierung notwendig sind. Vielmehr können andere Ausführungsbeispiele auch alternative Elemente und Komponenten, weniger Elemente oder Komponenten oder zusätzliche Elemente oder Komponenten enthalten. Elemente oder Komponenten verschiedener Ausführungsbespiele können miteinander kombiniert werden, sofern nichts anderes angegeben ist. Modifikationen und Abwandlungen, welche für eines der Ausführungsbeispiele beschrieben werden, können auch auf andere Ausführungsbeispiele anwendbar sein. Zur Vermeidung von Wiederholungen werden gleiche oder einander entsprechende Elemente in verschiedenen Figuren mit gleichen Bezugszeichen bezeichnet und nicht mehrmals erläutert. Von den Figuren zeigen:
- Fig. 1: eine perspektivische Ansicht eines Ausführungsbeispiels der erfindungsgemäßen Applizierungsvorrichtung 1;
- Fig. 2: eine schematische Schnittansicht der Applizierungsvorrichtung 1 von Fig. 1;
- Fig. 3: eine schematische vergrößerte Schnittansicht der ersten Frontgruppe 13;
- Fig. 4: eine schematische Schnittansicht der ersten Frontgruppe 13 sowie des distalen Endes 30 des Aufnahmeblocks 10 in einem nicht miteinander verbundenen Zustand;
- Fig. 5: eine schematische Schnittansicht der in das distale Ende des Aufnahmeblocks 10 eingeschraubten ersten Frontgruppe 13, wobei der Kolben 36 in seiner hinteren Endposition steht;
- Fig. 6: eine Schnittansicht gemäß Fig. 5, wobei der Kolben 36 in seiner vorderen Endposition und der Auslösekäfig 21 in seiner Freigabeposition für den Applizierungsvorgang steht;
- Fig. 7: eine perspektivische Darstellung des Aufnahmeblocks 10 samt Spannvorrichtung S, wobei die Spannvorrichtung S in der Grundstellung ist, in der sich der Kolben 36 in seiner vorderen Endposition befindet;
- Fig. 8: eine perspektivische Darstellung des Aufnahmeblocks 10 samt Spannvorrichtung S gemäß Fig. 7, wobei die Spannvorrichtung S in ihrer gespannten Stellung ist, in der sich der Kolben 36 in seiner hinteren Endposition befindet;
- Fig. 9: eine Schnittansicht des Aufnahmeblocks 10 samt Spannvorrichtung S gemäß Fig. 8;
- Fig. 10: eine perspektivische vergrößerte Detail-Darstellung des hinteren Teils der Spannvorrichtung S mit Walze 51 und Rampe 52 einer Abwandlung der erfindungsgemäßen Applizierungsvorrichtung 1 mit nur einer Zylinder-Kolben-Anordnung;
- Fig. 11A: ein Diagramm zur Darstellung des Verlaufs der Rampenbahn 53, wobei entlang der x-Achse der Drehwinkel und entlang der y-Achse der Hub entlang der Längsachse der Kolbenstange 36 aufgetragen ist,
- Fig. 11B: ein Diagramm zur Darstellung des abgewandelten Verlaufs der Rampenbahn 53, wobei entlang der x-Achse der Drehwinkel und entlang der y-Achse der Hub entlang der Längsachse der Kolbenstange 36 aufgetragen ist,
- Fig. 12: eine perspektivische Darstellung der Walze 51;
- Fig. 13: eine Draufsicht der Walze 51;
- Fig. 14 und 15: perspektivische Darstellungen des Aufnahmeblocks 10;
- Fig. 16: eine Draufsicht des Aufnahmeblocks 10, und
- Fig. 17: eine Schnittansicht des Aufnahmeblocks 10.

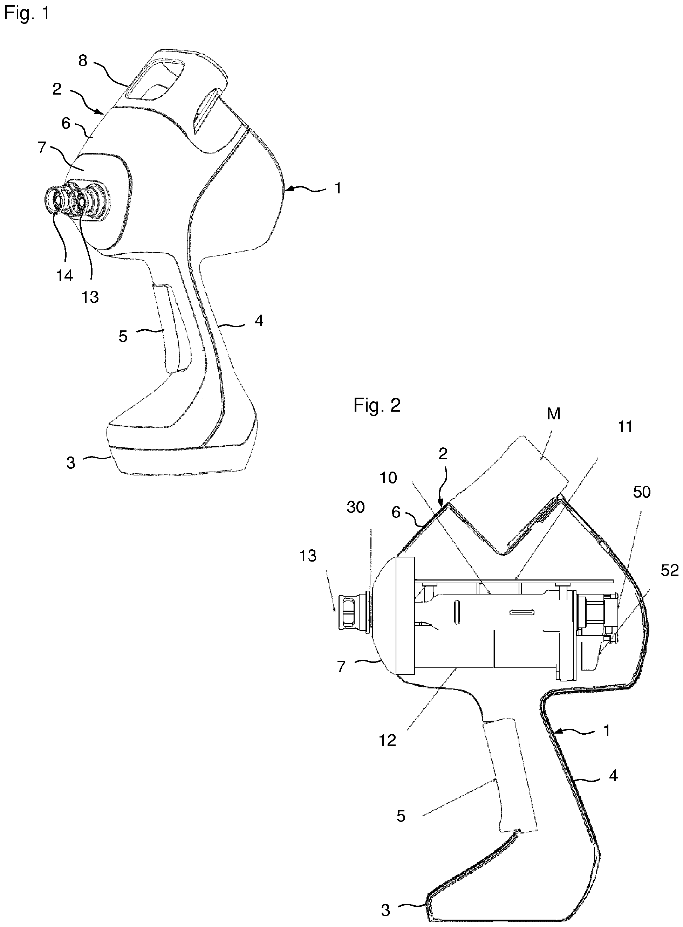

Bei dem in Fig. 1 gezeigten Ausführungsbeispiel umfasst die erfindungsgemäße Vorrichtung 1 zum Applizieren eines Fluids (z.B. einer Flüssigkeit) ein Gehäuse 2, das einen Fuß 3, der als

Standfuß 3 ausgebildet sein kann, einen Griffabschnitt 4 zum Halten der Vorrichtung 1, einen im Griffabschnitt 4 angeordneten Auslöser 5 zum Betätigen der Vorrichtung 1, einen Kopfbereich 6 mit einem Abgabebereich 7 sowie eine Aufnahme 8 im oberen Ende des Kopfbereiches 6 umfasst.

Die erfindungsgemäße Vorrichtung 1, die auch als Applizierungsvorrichtung 1 bezeichnet werden kann, ist bei dem hier beschriebenen Ausführungsbeispiel zum gleichzeitigen Applizieren von zwei unterschiedlichen Arzneimitteln bei Tieren ausgebildet, wobei die Applikation der Arzneimittel nadellos durch die Haut durchgeführt wird.

Bei der erfindungsgemäßen Applizierungsvorrichtung 1 ist für jedes Medikament, wie nachfolgend noch im Detail beschrieben wird, eine separate Zylinder-Kolben-Anordnung vorgesehen, die jeweils als Selbstfüllertyp derart ausgebildet ist, dass ein Bewegen des Kolbens zum Abgabeende hin ein Ausspritzen des Fluids bewirkt und eine entgegengesetzte Bewegung des Kolbens ein Auffüllen des Zylinders für den nächsten Ausspritzvorgang bewirkt.

Wie der schematischen Schnittdarstellung der Applizierungsvorrichtung 1 in Fig. 2 zu entnehmen ist, umfasst die Applizierungsvorrichtung 1 einen Aufnahmeblock 10, der eine Steuerungsplatine 11 sowie einen Motor 12 trägt, sowie zwei Frontgruppen 13, 14, von denen in der Darstellung von Fig. 2 nur die Frontgruppe 13 sichtbar ist. Da die Frontgruppen 13 und 14 baugleich ausgebildet sind, wird nachfolgend im Detail im Wesentlichen nur die Frontgruppe 13 beschrieben. In der Aufnahme 8 ist schematisch ein Medikamentenbehälter M, der ein flüssiges Arzneimittel für die Frontgruppe 13 enthält, eingezeichnet.

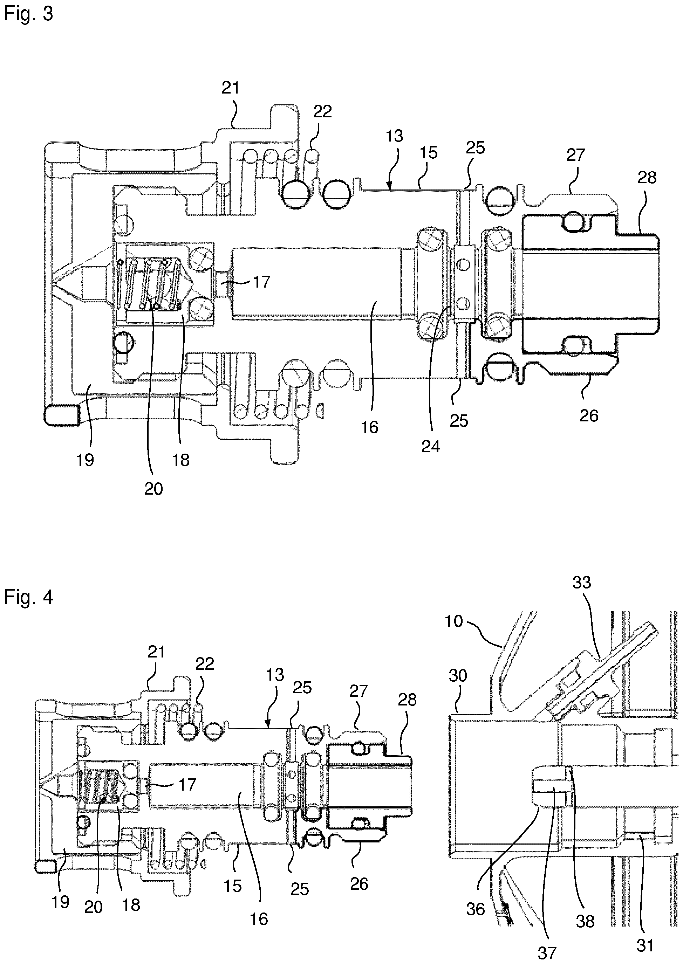

Eine Schnittdarstellung der Frontgruppe 13 ist in Fig. 3 gezeigt. Die Frontgruppe 13 umfasst einen Einsatz 15, in dem ein Spritzenzylinder 16 mit einem offenen Abgabeende 17 ausgebildet ist. Am offenen Abgabeende 17 ist austrittsseitig ein Rückschlagventil 18 (bzw. ein Ansaugventil 18) angeordnet. Das offene Abgabeende 17 mündet, bei geöffnetem Rückschlagventil 18, in eine Düse 19, über die das abzugebende Fluid (hier das entsprechende flüssige Arzneimittel) abgegeben wird.

Das Rückschlagventil 18 ist über eine Feder 20 zum offenen Abgabeende 17 hin vorgespannt und verschließt das offene Abgabeende 17 bei der in Fig. 3 gezeigten Stellung des Rückschlagventils 18.

Ferner umfasst die Frontgruppe 13 einen sich über die Düse 19 erstreckenden Auslösekäfig 21, der mittels einer Feder 22 in der Richtung vom offenen Abgabeende 17 zur Düse 19 hin gedrückt und vorgespannt wird. Der Auslösekäfig 21 ist entlang der Längsachse der Frontgruppe 13 (von links nach rechts in Fig. 3) verschiebbar gelagert, so dass er beim Ansetzen der Applizierungsvorrichtung 1 auf die entsprechende Hautstelle beim Tier in Richtung von der Düse 19 zum offenen Abgabeende 17 hin verschoben wird und dabei beispielsweise einen (nicht gezeigten) Kontaktsensor auslöst, der das Auslösen des Applikationsvorganges freigibt, wie nachfolgend noch im Detail beschrieben wird.

Der Einsatz 15 weist an einem dem offenen Abgabeende 17 gegenüberliegenden proximalen Ende 24 des Spritzenzylinders 16 radial verlaufende Zuführkanäle 25 auf, über die das zu applizierende Fluid bzw. das flüssige Arzneimittel für einen nächsten Ausspritzvorgang in den Spritzenzylinder 16 gelangt.

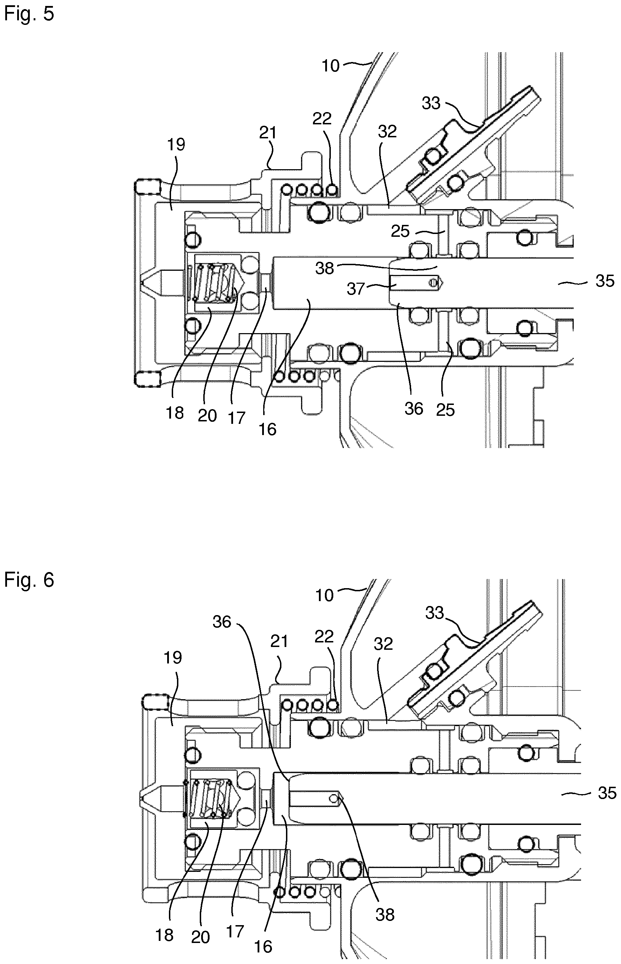

Am proximalen Ende 26 der Frontgruppe 13 ist ein Außengewinde 27 ausgebildet sowie eine Führungsbuchse 28 angeordnet, so dass die Frontgruppe 13 in ein distales Ende 30 des Aufnahmeblocks 10 (Fig. 4) eingeschraubt werden kann, da im distalen Ende 30 ein Innengewinde 31 für das Außengewinde 27 der Frontgruppe 13 vorgesehen ist. In Fig. 4 ist die Frontgruppe 13 und das distale Ende 30 des Aufnahmeblocks 10 vor dem Einschraubvorgang gezeigt. In Fig. 5 sind die beiden Elemente 13, 30 miteinander verschraubt, so dass eine im Aufnahmeblock 10 geführte Kolbenstange 35 mit einem an ihrem distalen Ende ausgebildeten Kolben 36 geringfügig in den Spritzenzylinder 16 einsteht und die Kolbenstange 35 durch die Führungsbuchse 28 geführt ist. Die Kolbenstange 35 kann, wie nachfolgend im Detail beschrieben wird, von der in Fig. 5 gezeigten Grundposition (nachfolgend auch Grundstellung genannt) zu der in Fig. 6 gezeigten Ausspritz- bzw. Abgabeposition (nachfolgend auch Ausspritz- bzw. Abgabestellung genannt) in Richtung zum offenen Abgabeende 17 hin bewegt werden und von dieser wieder zurück in die in Fig. 5 gezeigte Grundposition.

Steht die Kolbenstange 35 in der Grundposition steht, ist ihr distales Ende und somit der Kolben 36 in seiner hinteren Endposition (Fig. 5). Wenn die Kolbenstange 35 in der Abgabeposition steht, befindet sich der Kolben 36 in seiner vorderen Endposition (Fig. 6).

Wenn die Kolbenstange 35 in der in Fig. 5 gezeigten Grundposition positioniert ist, ist der Spritzenzylinder 16 mit dem auszuspritzenden flüssigen Arzneimittel gefüllt. Eine Bewegung der Kolbenstange 35 hin zum offenen Abgabeende 17 führt dann dazu, dass das Rückschlagventil 18 öffnet und so die Flüssigkeit über die Düse 19 als Strahl abgegeben wird, der so weit in die Haut des Tieres einschneidet, dass das Arzneimittel durch diesen Schnitt in die Haut appliziert werden kann.

Bei der Rückwärtsbewegung von der in Fig. 6 gezeigten Abgabestellung zu der in Fig. 5 gezeigten Grundstellung (eine Bewegung entlang einer ersten Richtung) schließt das Rückschlagventil 18 und wird im Spritzenzylinder 16 ein Unterdruck erzeugt, der umso größer wird, je weiter die Kolbenstange 35 vom offenen Abgabeende 17 weg bewegt wird. Sobald die Kolbenstange 35 in ihre Grundposition gebracht ist, liegt eine Fluidverbindung zwischen dem Spritzenzylinder 16 und mindestens einem der Zuführkanäle 25 vor. Um die Fluidverbindung zu realisieren, ist eine axiale Sackbohrung 37 im distalen Ende des Kolbens 36 und mindestens eine sich von der Sackbohrung 37 radial erstreckende Querbohrung 38, deren der Sackbohrung 37 abgewandtes Ende in einem der Zuführkanäle 25 mündet, ausgebildet. Da die Zuführkanäle 25 ihrerseits in eine zwischen dem Einsatz 15 und dem distalen Ende 30 des Aufnahmeblocks 10 gebildete Kammer 32 münden, die über ein Anschlusselement 33 mit dem nur in Fig. 2 gezeigten Medikamentenbehälter M verbunden ist, wird das flüssige Arzneimittel aufgrund des im Spritzenzylinder 16 vorhandenen Unterdrucks aus dem Medikamentenbehälter M über das Anschlusselement 33, die Kammer 32, den bzw. die Zuführkanäle 25, die Querbohrung(en) 38 und die Sackbohrung 37 in den Spritzenzylinder 16 gesaugt, so dass dieser mit dem flüssigen Arzneimittel gefüllt wird. Somit kann beim nächsten Auslösevorgang erneut das Arzneimittel appliziert werden, wobei zu Beginn der Bewegung der Kolbenstange 35 von der Grundposition zur Ausspritzposition etwas vom flüssigen Arzneimittel über die Zuführkanäle 25 in die Kammer 32 zurückgedrückt wird. Dieses Zurückdrücken ist vorteilhaft, da dadurch der Kolben 36 bzw. die Kolbenstange 35 leichter beschleunigt werden kann, was zu einem höheren Druck führt, mit dem das restliche Arzneimittel im Spritzenzylinder 16 beaufschlagt wird und der für die beschriebene nadellose Injektion gewünscht ist.

Die Frontgruppe 13, die insbesondere den Spritzenzylinder 16, das Rückschlagventil 18 sowie die Düse 19 umfasst, ist als Ganzes austauschbar ausgebildet. Sie kann mittels ihrem Außengewinde 27 in das entsprechende Innengewinde 31, das am distalen Ende 30 des Aufnahmeblocks 10 ausgebildet ist, eingeschraubt und wieder ausgeschraubt werden. Nachdem die Frontgruppe 13 einem Verschleiß im Betrieb der Applizierungsvorrichtung 1 unterliegt, kann somit eine abgenutzte Frontgruppe 13 leicht gegen eine neue baugleiche Frontgruppe 13 ausgetauscht werden. Dies führt vorteilhaft dazu, dass die Applizierungsvorrichtung 1 insgesamt länger benutzt werden kann, da die verschleißanfälligsten Bestandteile der Applizierungsvorrichtung 1 leicht ausgetauscht werden können.

Da die Frontgruppe 13 komplett austauschbar ist, können unerwünschte Kontaminationen sicher vermieden werden, was deutlich schwieriger zu vermeiden und mit höheren Kosten verbunden wäre, wenn z.B. die zuerst verschleißenden O-Ring-Dichtungen bei aus dem Stand bekannten Applizierungsvorrichtungen im Bereich des Zylinders, der fest und nicht austauschbar mit der restlichen aus dem Stand bekannten Vorrichtung verbunden ist, einzeln ausgetauscht werden würden.

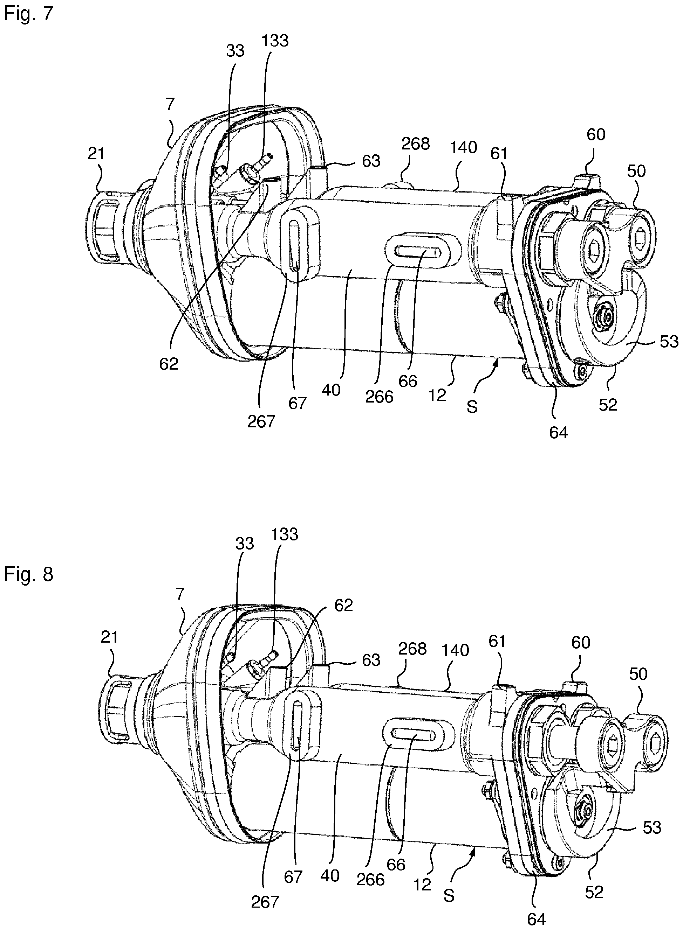

Wie am besten aus Fig. 7 bis 9 ersichtlich ist, weist der Aufnahmeblock 10 einen ersten Aufnahmezylinder 40, in den die Kolbenstange 35 für die erste Frontgruppe 13 geführt ist, sowie einen zweiten Aufnahmezylinder 140, in dem eine Kolbenstange 135 für die zweite Frontgruppe 14 geführt ist, auf. Da der Aufbau der beiden Zylinder-Kolben-Anordnungen und somit auch der beiden Aufnahmezylinder 40, 140 gleich ist, wird nachfolgend bei der Beschreibung des Aufnahmeblocks 10 im Wesentlichen nur die erste Zylinder-Kolben-Anordnung mit dem ersten Aufnahmezylinder 40 beschrieben. Entsprechende Elemente beim zweiten Aufnahmezylinder 140 werden mit Bezugszeichen bezeichnet, die um 100 größer sind als bei den Elementen des ersten Aufnahmezylinders 40, werden aber nicht nochmal beschrieben.

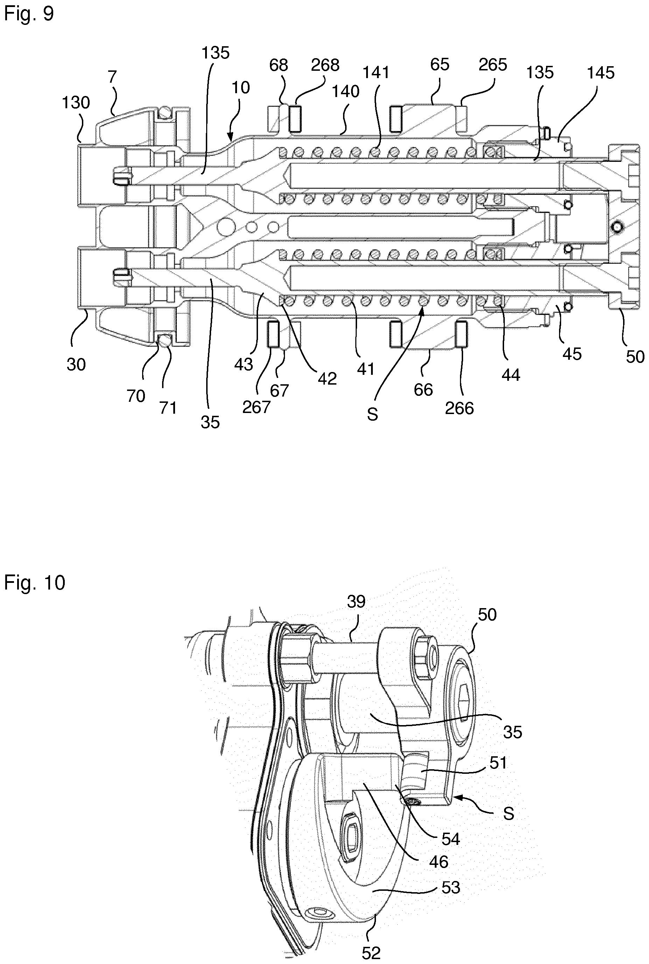

Die Kolbenstange 35 verläuft durch den ersten Aufnahmezylinder 40, in dem eine Feder 41 zur Bewegung der Kolbenstange 35 von der in Fig. 5 gezeigten gespannten Grundposition zu der in Fig. 6 gezeigten Ausspritzposition enthalten ist. Ein distales Ende 42 der Feder 41 liegt an einem Anschlagabschnitt 43 der Kolbenstange 35 an. Das proximale Ende 44 der Feder 41 liegt an einer Führungsbuchse 45, die in das proximale Ende des ersten Aufnahmezylinders 40 eingeschraubt ist, an, so dass bei einer Bewegung der Kolbenstange von der in Fig. 6 gezeigten Ausspritzpositionen entlang der ersten Richtung zu der in Fig. 5 gezeigten vorgespannten Grundposition die Feder 41 komprimiert und dadurch vorgespannt wird, wie dies in Fig. 9 dargestellt ist.

Wie insbesondere aus der vergrößerten perspektivischen Darstellung in Fig. 10 zu entnehmen ist, ist das aus dem Aufnahmezylinder 40 proximal herausstehende Ende der Kolbenstange 35 mit einem Mitnehmer 50 verbunden, der eine drehbar gelagerte Walze 51 aufweist, wobei sich die Drehachse der Walze 51 im Wesentlichen senkrecht zur Längsachse der Kolbenstange 35 erstreckt.

Bei der perspektivischen Darstellung in Fig. 10 ist eine Abwandlung der erfindungsgemäßen Applizierungsvorrichtung 1 gezeigt. Bei dieser Abwandlung ist nur eine einzige Zylinder-Kolben-Anordnung ausgebildet und der Mitnehmer 50 ist mit dem proximalen Ende der Kolbenstange 35 sowie dem proximalen Ende einer Führungsstange 39, die im Aufnahmeblock 10 verschiebbar gelagert ist, verbunden.

Die Walze 51 läuft auf einer sich unter der Walze 51 drehenden Rampe 52, die durch den Motor 12 um eine Achse parallel zur Längsachse der Kolbenstange 35 gedreht wird. Als Stromversorgung für den Motor kann eine Batterie und/oder ein Akkumulator, der wiederaufladbar ist, vorgesehen sein. Die Batterie und/oder der Akkumulator kann z.B. im Fuß 3 oder als Fuß 3 der Applizierungsvorrichtung 1 angeordnet sein.

Die Rampe 52 weist eine Rampenbahn 53 auf, die entlang einer Schraubenlinie mit einer einzigen Windung verläuft, wie insbesondere Fig. 7, 8 und 10 zu entnehmen ist.

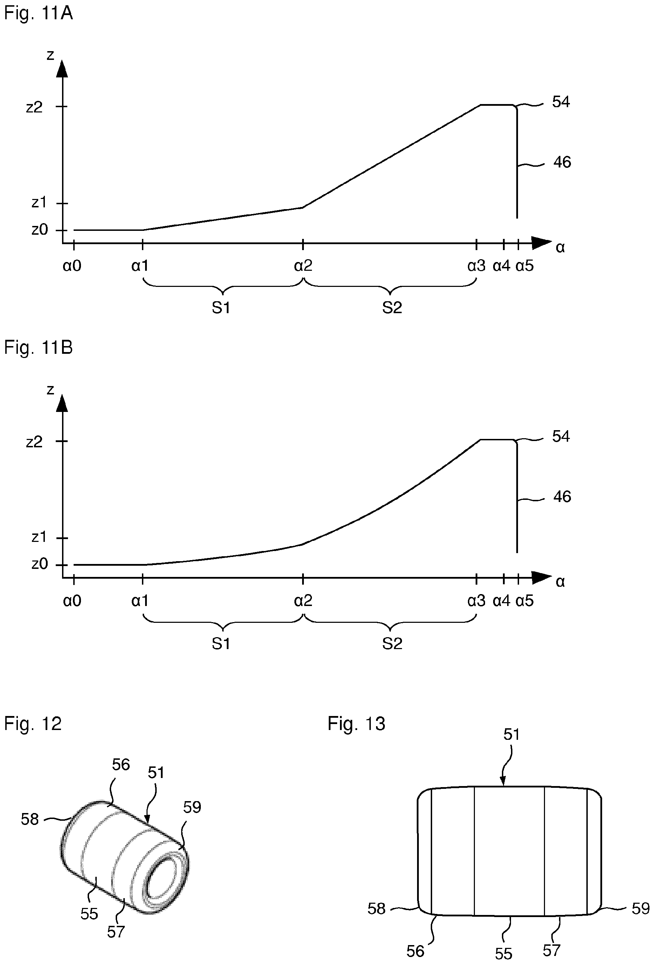

In Fig. 11A ist der Drehwinkel α gegenüber dem Gangunterschied z parallel zur Längsrichtung der Kolbenstange 35 aufgetragen, wobei davon ausgegangen wird, dass beim Drehwinkel von α0 = 0° die geringste Ganghöhe z0 vorliegt und der Kolben 36 in seiner vorderen Endposition steht. Wird nun die Rampe 52 gedreht, läuft die Rampenbahn 53 unter der Walze 51 durch und führt dazu, dass die Drehbewegung der Rampenbahn 53 in eine translatorische Bewegung der Walze 51 samt Mitnehmer 50 und somit der Kolbenstange 35 entlang der Längsachse der Kolbenstange 35 umgesetzt wird, so dass die Kolbenstange 35 von ihrer in Fig. 6 gezeigten Ausspritzposition zu der in Fig. 5 gezeigten Grundposition bewegt wird.

Dazu verläuft die abgewickelte Rampenbahn 53 gemäß Fig. 11A so, dass vom Drehwinkel α0 bis zu einem Drehwinkel α1 ein erstes Plateau (Steigung = 0 oder nur etwas größer oder etwas kleiner als Null) vorliegt. Ab dem Drehwinkel α1 weist die Rampenbahn 53 einen ersten linearen Steigungsabschnitt S1 auf, die bis zum Drehwinkel α2 vorliegt. Beim Drehwinkel α2 (und der entsprechenden Ganghöhe z1) geht die Steigung in einen zweiten linearen Steigungsabschnitt S2 über, der eine größere Steigung als die erste lineare Steigungsabschnitt S1 (zwischen den Drehwinkeln α1 und α2) aufweist. Die zweite Steigung liegt bis zum Drehwinkel α3 vor und geht danach in ein zweites Plateau über, in dem keine Zunahme (oder nur eine sehr geringe Zunahme oder nur eine sehr geringe Abnahme) der Ganghöhe z2 bei einer weiteren Zunahme des Drehwinkels bis zum Drehwinkel a4 von kleiner als 360° erfolgt.

Beim Drehwinkel α5 ist aufgrund der Sprungflanke 46, die das zweite Plateau mit dem ersten Plateau verbindet, eine Kante 54 ausgebildet.

In dem Bereich von a3 bis a4 ist die Kolbenstange 35 in ihrer gespannten Grundposition gemäß Fig. 5. Die Applizierungsvorrichtung 1 ist somit bereit für die Applikation des flüssigen Arzneimittels.

Wird nun der Auslöser 5 betätigt und steht der Auslösekäfig 21 in der Auslöseposition, dreht der Motor 12 die Rampe 52 weiter, so dass beim Überschreiten des Drehwinkels α5 die Walze 51 über die Kante 54 läuft und aufgrund der Federspannung der Feder 41 schlagartig von der Ganghöhe z2 auf die Ganghöhe z0 des ersten Plateaus zurückfällt, so dass das in dem Spritzenzylinder 16 vorhandene Fluid in der beschriebenen Art und Weise ausgespritzt wird.

Der Motor 12 dreht die Rampe 52 danach weiter bis zum zweiten Plateau und stoppt hier die Drehbewegung, so dass dadurch der Spritzenzylinder 16 erneut mit dem flüssigen Arzneimittel gefüllt wird und die Kolbenstange 35 wieder in ihre gespannte Grundposition gebracht ist. Somit ist die Applizierungsvorrichtung 1 für einen weiteren Applikationsvorgang bereit. In dieser Art und Weise kann die Applizierungsvorrichtung 1 wiederholt aufgezogen und ausgelöst werden.

Die beiden Steigungsabschnitte S1 und S2 bilden einen Steigungsbereich, der vom ersten Plateau bis zum zweiten Plateau verläuft. Die beiden Steigungsabschnitte S1 und S2 müssen nicht linear bezogen auf den Drehwinkel verlaufen. Sie können auch, wie beispielhaft in Fig. 11B gezeigt ist, eine konkave Krümmung (bei der die lokale Steigung mit zunehmenden Drehwinkel zunimmt) aufweisen. Jedoch ist auch in diesem Fall die mittlere Steigung des ersten Steigungsabschnitts S1 kleiner als die mittlere Steigung des zweiten Steigungsabschnitts S2.

Der Kombination aus Motor 12, Rampe 52, Mitnehmer 50 mit Walze 51, Feder 41, 141 und Führungsbuchse 45, 145 kann als Spannvorrichtung S bezeichnet werden.

Wie insbesondere Fig. 12 und 13 zu entnehmen ist, umfasst die Walze 51 einen Mittelbereich 55, der einen konstanten Außendurchmesser aufweist. Daran schließt sich zu beiden Seiten jeweils ein Seitenbereich 56, 57 an, in dem der Außendurchmesser zur Seite hin abnimmt.

An beide Seitenbereiche 56 und 57 schließt sich jeweils ein Endbereich 58, 59 an, der abgerundet ist, damit die Walze 51 keine Kanten aufweist. Der Mittelbereich 55 liegt bei Drehung der Rampe 52 auf der Rampenbahn 53 im Drehwinkelbereich von α1 bis α3 (insbesondere von α0 bis α5) auf, wohingegen die Seitenbereiche 56 und 57 in diesem Drehwinkelbereich nicht aufliegen, sondern nur beim Überlaufen der Kante 54 in Kontakt mit der Rampenbahn 53 kommen . Somit kann der Rollwiderstand der Walze 51 beim Spannen der Kolbenstange 35 (Drehen der Rampenbahn vom ersten zum zweiten Plateau) möglichst gering ist. Beim Übergang vom zweiten bzw. oberen Plateau (Drehwinkelbereich a3 bis a4) der Rampenbahn 53 zum unteren Plateau (Drehwinkelbereich α1 bis α2) sind auch die Seitenbereiche 56 und 57 in Kontakt mit der Kante 54, wodurch sich in vorteilhafter Weise die Kräfte zwischen der Walze 51 und der Kante 54 der Rampenbahn 52 auf eine größere Auflagefläche verteilen (Mittelbereich 55 sowie die beiden Seitenbereiche 56, 57), so dass ein kleinerer Druck vorliegt. Dadurch wird die Haltbarkeit der Vorrichtung 1 und insbesondere der Walze 51 erhöht.

Die in Verbindung mit Fig. 11A und 11B beschriebene Charakteristik der Steigung der Rampenbahn 53 ist von Vorteil, da zu Beginn (Drehwinkelbereich α1 bis α2) eine kleinere Steigung vorliegt, so dass weniger Drehmomente durch den Motor 50 bereitgestellt werden muss. Dies ist gerade beim Anlaufen des Motors 50 ausgehend vom ersten Plateau von Vorteil, da der Motor 50 in diesem Bereich üblicherweise mehr Strom zieht. Wenn die Ganghöhe z1 erreicht ist, kann die höhere Steigung im Drehwinkelbereich von a2 bis a3 problemlos vom Motor 50 bewältigt werden.

Diese Charakteristik der Steigung der Rampenbahn 52 führt in vorteilhafter Weise dazu, dass die Haltbarkeit des Motors 50 erhöht ist.

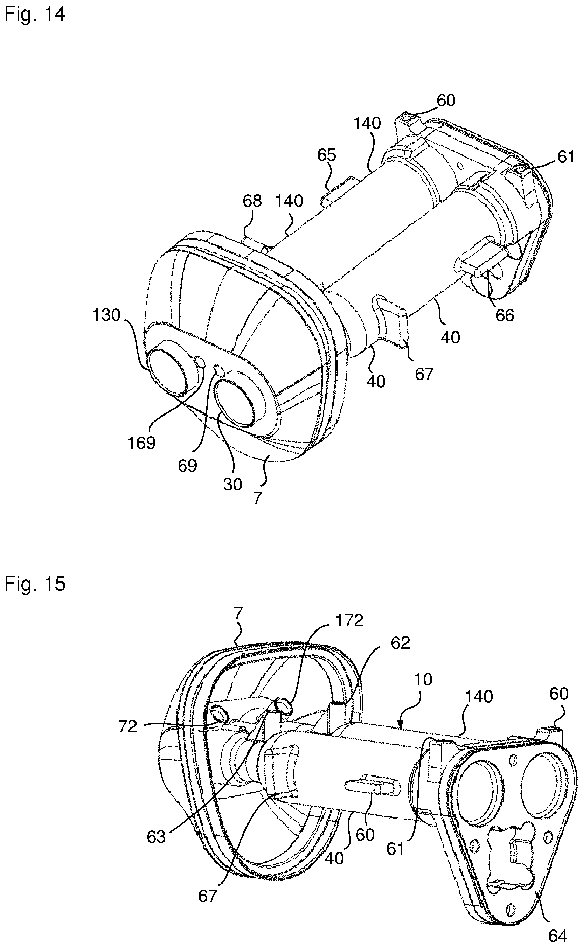

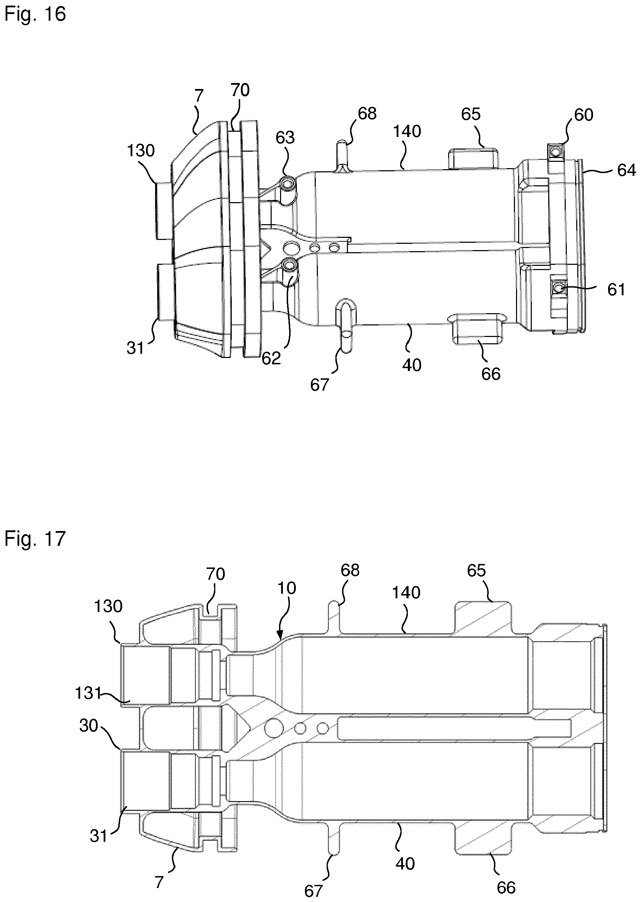

Wie am besten aus den Darstellungen von Fig. 14 bis 17 zu entnehmen ist, ist der Aufnahmeblock 10 einstückig ausgebildet. Dabei wurde hier beispielsweise ein additives Herstellungsverfahren eingesetzt, wie z.B. Lasersintern, selektives Lasersintern oder direktes Metall-Lasersintern, mit dem feinere und/oder komplexere Strukturen im Vergleich zu spanabhebenden Verfahren erzeugt werden können. Der Aufnahmeblock 10 kann somit mit einem relativ geringen Gewicht bereitgestellt werden und es kann eine große Haltbarkeit der Applizierungsvorrichtung 1 gewährleistet werden.

Als Material für den Aufnahmeblock 10 kann z.B. Aluminium, Stahl (z.B. Maraging-Stahl), Edelstahl, Titan, eine Nickel-Legierung und/oder eine Kobalt-Chrom-Legierung verwendet werden. Dabei liegt das Material für das Lasersintern bevorzugt als Metallpulver vor. Zur additiven bzw. schichtweisen Herstellung des Aufnahmeblocks 10 kann eine dünne Schicht des Pulverwerkstoffs auf eine Bauplattform aufgetragen werden. Ein Laserstrahl schmilzt das Pulver exakt an den Stellen auf, die von computergenerierten Bauteil-Konstruktionsdaten des Aufnahmeblocks 10 vorgegeben sind. Danach wird die Bauplattform abgesenkt und es erfolgt der Auftrag einer weiteren dünnen Schicht des Pulverwerkstoffs. Der Werkstoff wird erneut aufgeschmolzen und verbindet sich an den definierten Stellen mit der darunterliegenden Schicht. Diese Schritte werden so lange wiederholt, bis der gesamte Aufnahmeblock 10 gebildet ist.

Der Aufnahmeblock 10 umfasst neben den bereits beschriebenen Aufnahmezylindern 40, 140 vier Platinenaufnahmestellen 60, 61, 62 und 63, auf die die Platine 11 aufgesetzt und mit dem Aufnahmeblock 10 beispielsweise verschraubt werden kann.

Ferner umfasst der Aufnahmeblock ein Motorlager 64 zur Aufnahme und Lagerung des Motors 12.

Des Weiteren sind vier Fixationsstellen 65, 66, 67 und 68 für das Außengehäuse 2 der Applizierungsvorrichtung 1 ausgebildet. Abschnitte von entsprechenden Aufnahmen 265, 266, 267 und 268 des Außengehäuses 2 sind in Fig. 7 bis 9 eingezeichnet.

Am distalen Ende des Aufnahmeblocks 10 ist der schirmartige Abgabebereich 7 vorgesehen, der neben dem entsprechenden Innengewinde 31 und 131 für die erste und zweite Frontgruppe 13 und 14 noch Aufnahmen 69 und 169 für den jeweiligen Auslösesensor (nicht gezeigt), der die Stellung des Auslösekäfigs 21, 121 detektiert, aufweist.

Des Weiteren ist im Abgabebereich 7 eine O-Ring-Aufnahme 70 ausgebildet, in die ein O-Ring 71 (z.B. Fig. 9, 16 und 17) eingesetzt werden kann, um eine Abdichtung gegenüber dem anliegenden Gehäuse 2 im eingebauten Zustand zu gewährleisten.

Ferner umfasst der Aufnahmeblock 10 für jeden Aufnahmezylinder 40, 140 eine Aufnahme 72, 172, in die das entsprechende Anschlusselement 33, 133, das auch als Fluidadapter bezeichnet werden kann, eingesetzt werden kann.

Das Anschlusselement 33, 133 ist bevorzugt zerspanend hergestellt.

Als Material für den Aufnahmeblock 10 wird bevorzugt Titan eingesetzt. Dieses Material ist einerseits relativ leicht und gewährleistet andererseits die gewünschte Festigkeit. Es kann natürlich jedes andere geeignete Material für additive Herstellungsverfahren verwendet werden.

Bei der bisherigen Beschreibung wurde davon ausgegangen, dass zwei unterschiedliche Medikamente gleichzeitig appliziert werden sollen. Die erfindungsgemäße Applizierungsvorrichtung 1 kann jedoch auch so ausgebildet sein, dass der Aufnahmeblock 10 nur einen einzelnen Aufnahmezylinder 40 aufweist, so dass auch nur ein einziges Medikament bei einem Auslösevorgang appliziert werden kann. Der zweite Aufnahmezylinder 140 sowie die zweite Zylinder-Kolbenstange-Kombination sind dann bevorzugt weggelassen.

Bei den bisher beschriebenen Ausführungsbeispielen wurde davon ausgegangen, dass die Applizierungsvorrichtung 1 als nadellose Applizierungsvorrichtung 1 ausgebildet ist. Sie kann jedoch auch als Applizierungsvorrichtung 1 mit einer Nadel bzw. einer Kanüle ausgebildet sein, so dass in diesem Fall die Nadel bzw. Kanüle in die Haut des Tieres einzustechen ist und danach die Beaufschlagung mittels des flüssigen Arzneimittels in der beschriebenen Art und Weise erfolgt.

## Patentansprüche

1. Vorrichtung zum nadellosen Applizieren eines Fluids, mit
einem Zylinder (16, 116), der ein offenes Abgabeende (17) aufweist,
einem im Zylinder (16, 116) zwischen einer vorderen und hinteren Endposition verschiebbaren Kolben (36), der mit einer Kolbenstange (35) verbunden ist, die entlang einer ersten Richtung über ein dem offenen Abgabeende (17) entgegengesetztes hinteres Ende des Zylinders (16, 116) heraussteht und in einem Aufnahmeblock (10) geführt ist,
einem das offene Abgabeende (17) verschließenden Rückschlagventil (18) und
einer mit der Kolbenstange (35, 135) verbundenen Spannvorrichtung (S), die im Aufnahmeblock (10) angeordnet ist,
wobei die Spannvorrichtung (S), wenn der Kolben (36) in seiner vorderen Endposition steht, die Kolbenstange (35, 135) in einem Spannvorgang entlang der ersten Richtung bewegen kann, bis der Kolben (36) in seiner hinteren Endposition steht, um den Zylinder (16, 116) dadurch mit dem zu applizierenden Fluid zu füllen und die Kolbenstange (35, 135) zum offenen Abgabeende (17) hin vorzuspannen, und
wobei die Spannvorrichtung (S), wenn der Kolben (36) in seiner hinteren Endposition steht, die Kolbenstange (35, 135) in einem Abgabevorgang freigeben kann, so dass der Kolben (36) aufgrund der anliegenden Vorspannung entgegen der ersten Richtung bis zu seiner vorderen Endposition bewegt und dabei Fluid im Zylinder (16, 116) über das Rückschlagventil (18) zum Applizieren abgegeben wird,
wobei der Zylinder (16, 116) zusammen mit dem Rückschlagventil (18) als austauschbare Frontgruppe (13, 14) ausgebildet ist, die mit dem Aufnahmeblock (10) lösbar verbunden ist,
und wobei die Vorrichtung eine Düse (19) zum nadellosen Applizieren des Fluids aufweist, die über das Rückschlagventil (18) mit dem offenen Abgabeende des Zylinders (16, 116) verbunden ist und die Bestandteil der Frontgruppe (13, 14) ist.

2. Vorrichtung nach Anspruch 1, wobei
die lösbare Verbindung zwischen Frontgruppe (13, 14) und Aufnahmeblock (10) eine Schraubverbindung ist.

3. Vorrichtung nach einem der obigen Ansprüche, wobei
die Spannvorrichtung (S) eine Feder (41, 141) aufweist, die Kolbenstange (35, 135) zum offenen Abgabeende hin vorspannt, wenn der Kolben (36) in der hinteren Endposition steht.

4. Vorrichtung nach einem der obigen Ansprüche, wobei
die Spannvorrichtung (S) eine mittels eines Motors (12) drehbare Rampe (52) mit einer sich entlang einer Schraubenlinie erstreckenden Rampenbahn (53) aufweist,
wobei die Rampenbahn (53) von einem ersten Plateau entlang eines Steigungsbereiches (S1, S2) zu einem zweiten Plateau ansteigt und vom zweiten Plateau über eine Sprungflanke (46) zum ersten Plateau abfällt,
wobei die Spannvorrichtung (S) ferner eine die Rampenbahn (53) kontaktierende Walze (51), die in einem Mitnehmer (50), der mit dem aus dem Zylinder (16, 116) herausstehenden Ende der Kolbenstange (35, 135) verbunden ist, drehbar gelagert ist, aufweist, so dass bei Drehung der Rampe (52) die Rampenbahn (53) unter der sich dadurch drehenden Walze (51) durchläuft,
wobei für den Spannvorgang die Rampenbahn (53) ausgehend von einem Kontakt der Walze (51) mit dem ersten Plateau so gedreht wird, dass die Walze (51) auf dem Steigungsbereich bis zum zweiten Plateau läuft und dadurch der Kolben (36) in seine hintere Endposition bewegt wird,
wobei für den Abgabevorgang die Rampenbahn (53) ausgehend von einem Kontakt der Walze (51) mit dem zweiten Plateau so lange gedreht wird, bis die Walze (51) über die Sprungflanke (46) auf das erste Plateau trifft und dadurch der Kolben (36) in seine vordere Endposition bewegt wird.

5. Vorrichtung nach Anspruch 4, wobei
der Motor (12) im Aufnahmeblock (10) gelagert ist.

6. Vorrichtung nach Anspruch 4 oder 5, wobei
der Steigungsbereich der Rampenbahn (53) einen an das erste Plateau anschließenden ersten Abschnitt (S1) und einen daran anschließenden zweiten Abschnitt (S2) aufweist, wobei die Steigung des zweiten Abschnitts (S2) größer ist als die Steigung des ersten Abschnitts (S1).

7. Vorrichtung nach Anspruch 6, wobei
beide Abschnitte (S1, S2) bezogen auf den Drehwinkel der Schraubenlinie linear verlaufen.

8. Vorrichtung nach Anspruch 5 oder 6, wobei der Drehwinkellänge des ersten Abschnitts (S1) kleiner ist als der Drehwinkellänge des zweiten Abschnitts (S2).

9. Vorrichtung nach einem der Ansprüche 4 bis 8, wobei
die Walze (51) einen Auflagebereich (55), der auf dem Steigungsbereich (S1, S2) der Rampenbahn (53) aufliegt, und mindestens einen sich daran seitlich anschließenden Seitenbereich (56, 57), der einen geringeren Außendurchmesser als der Auflagebereich (55) aufweist und der nicht auf dem Steigungsbereich (S1, S2) der Rampenbahn (53) aufliegt, aufweist,
wobei beim Abgabevorgang sowohl der Auflagebereich (55) als auch der Seitenbereich (56, 57) in Kontakt mit einer das zweite Plateau mit der Sprungflanke (46) verbindenden Kante (54) der Rampenbahn (53) kommt.

10. Vorrichtung nach Anspruch 9, wobei
die Walze (51) auf beiden Seiten des Auflagebereiches (55) je einen seitlich anschließenden Seitenbereich (56, 57) mit geringerem Außendurchmesser als der des Auflagebereichs (55) aufweist.

11. Vorrichtung nach Anspruch 9 oder 10, wobei
der Außendurchmesser des jeweiligen Seitenbereichs (56, 57) in einer Richtung zur Seite der Walze (51) hin abnimmt.

12. Vorrichtung nach einem der Ansprüche 4 bis 11, wobei
die Walze (51) im Mitnehmer (50) so gelagert ist, dass ihre Drehachse senkrecht zur ersten Richtung ist.

13. Vorrichtung nach einem der obigen Ansprüche, bei der
der Aufnahmeblock (10) als einstückiger Aufnahmeblock (10) ausgebildet ist, der durch ein additives Herstellungsverfahren hergestellt ist.

14. Vorrichtung nach Anspruch 13, wobei
der Aufnahmebock (10) als Metall-Aufnahmeblock (10) ausgebildet ist.

15. Vorrichtung nach Anspruch 13 oder 14, wobei
der Aufnahmeblock (10) ein Motorlager (64), einen Führungszylinder (40, 140) für die Kolbenstange (35, 135), mindestens eine Aufnahme (60, 61, 62, 63) für eine Steuerungsplatine (11), eine Aufnahme (72, 172) für einen Fluidanschluss (33) für einen Fluidbehälter (M) und/oder mindestens eine Gehäusefixierstelle (65, 66, 67, 68) aufweist, die einstückig mit dem Aufnahmeblock (10) ausgebildet ist/sind.

## Claims

1. Device for administering a fluid without a needle, comprising
a cylinder (16, 116) which has open dispensing end (17) ,
a piston (36) which is displaceable between a front and rear end position in the cylinder (16, 116) and is connected to a piston rod (35) that protrudes along a first direction beyond a rear end of the cylinder (16, 116) opposite the open dispensing end (17) and is guided in a receiving block (10),
a nonreturn valve (18) closing the open dispensing end (17), and
a tensioning device (S) which is connected to the piston rod (35, 135) and is arranged in the receiving block (10),
wherein the tensioning device (S), when the piston (36) is in its front end position, can move the piston rod (35, 135) in a tensioning operation along the first direction until the piston (36) is in its rear end position in order thereby to fill the cylinder (16, 116) with the fluid to be administered and in order to pretension the piston rod (35, 135) towards the open dispensing end (17), and wherein the tensioning device (S), when the piston (36) is in its rear end position, can release the piston rod (35, 135) in a dispensing operation, and therefore the piston (36) moves counter to the first direction as far as its front end position because of the applied pretension and, in the process, fluid in the cylinder (16, 116) is dispensed via the nonreturn valve (18) for administering,
wherein the cylinder (16, 116) together with the nonreturn valve (18) is in the form of an exchangeable front assembly (13, 14) which is releasably connected to the receiving block (10), and wherein the device has a nozzle (19) for administering the fluid without a needle, said nozzle being connected to the open dispensing end of the cylinder (16, 116) via the nonreturn valve (18) and being part of the front assembly (13, 14).

2. Device according to Claim 1, wherein
the releasable connection between the front assembly (13, 14) and the receiving block (10) is a screw connection.

3. Device according to either of the above claims, wherein
the tensioning device (S) has a spring (41, 141) which pretensions the piston rod (35, 135) towards the open dispensing end when the piston (36) is in the rear end position.

4. Device according to one of the above claims, wherein the tensioning device (S) has a ramp (52) which is rotatable by means of a motor (12) and has a ramp track (53) extending along a helical line,
wherein the ramp track (53) ascends from a first plateau along a region of inclination (S1, S2) to a second plateau and descends from the second plateau to the first plateau via a transition flank (46), wherein the tensioning device (S) furthermore has a roller (51) which is in contact with the ramp track (53) and is mounted rotatably in a driver (50), which is connected to that end of the piston rod (35, 135) which protrudes out of the cylinder (16, 116), and therefore, upon rotation of the ramp (52), the ramp track (53) runs below the roller (51), which thereby rotates,
wherein, for the tensioning operation, the ramp track (53), starting from a contact of the roller (51) with the first plateau, is rotated in such a manner that the roller (51) runs on the region of inclination as far as the second plateau and the piston (36) is thereby moved into its rear end position,
wherein, for the dispensing operation, the ramp track (53), starting from a contact of the roller (51) with the second plateau, is rotated until the roller (51) via the transition flank (46) reaches the first plateau and the piston (36) is thereby moved into its front end position.

5. Device according to Claim 4, wherein
the motor (12) is mounted in the receiving block (10) .

6. Device according to Claim 4 or 5, wherein
the region of inclination of the ramp track (53) has a first portion (S1) adjoining the first plateau and an adjoining second portion (S2), wherein the inclination of the second portion (S2) is greater than the inclination of the first portion (S1).

7. Device according to Claim 6, wherein
both portions (S1, S2) run linearly with respect to the angle of rotation to the helical line.

8. Device according to Claim 5 or 6, wherein the length of the angle of rotation of the first portion (S1) is smaller than the length of the angle of rotation of the second portion (S2).

9. Device according to one of Claims 4 to 8, wherein the roller (51) has a support region (55) which rests on the region of inclination (S1, S2) of the ramp track (53), and at least one laterally adjoining side region (56, 57) which has a smaller outside diameter than the support region (55) and which does not rest on the region of inclination (S1, S2) of the ramp track (53),
wherein, during the dispensing operation, both the support region (55) and the side region (56, 57) come into contact with an edge (54) of the ramp track (53), said edge connecting the second plateau to the transition flank (46).

10. Device according to Claim 9, wherein
the roller (51) on either side of the support region (55) has a laterally adjoining side region (56, 57) with a smaller outside diameter than that of the support region (55).

11. Device according to Claim 9 or 10, wherein
the outside diameter of the respective side region (56, 57) decreases in a direction towards the side of the roller (51).

12. Device according to one of Claims 4 to 11, wherein the roller (51) is mounted in the driver (50) in such a manner that the axis of rotation of said roller is perpendicular to the first direction.

13. Device according to one of the above claims, in which
the receiving block (10) is in the form of an integral receiving block (10) which is produced by an additive production method.

14. Device according to Claim 13, wherein
the receiving block (10) is in the form of a metal receiving block (10).

15. Device according to Claim 13 or 14, wherein
the receiving block (10) has a motor bearing (64), a guide cylinder (40, 140) for the piston rod (35, 135), at least one receptacle (60, 61, 62, 63) for a control board (11), a receptacle (72, 172) for a fluid connection (33) for a fluid container (M) and/or at least one housing fixing point (65, 66, 67, 68) which are/is formed integrally with the receiving block (10).

## Revendications

1. Dispositif pour l'application sans aiguille d'un fluide, pourvu
d'un cylindre (16, 116), qui présente une extrémité de distribution ouverte (17),
d'un piston (36) pouvant coulisser dans le cylindre (16, 116) entre une position d'extrémité avant et une position d'extrémité arrière, qui est relié à une tige de piston (35), qui dépasse, le long d'une première direction, d'une extrémité arrière, opposée à l'extrémité de distribution ouverte (17), du cylindre (16, 116) et qui est guidée dans un bloc de réception (10) ,
d'un clapet antiretour (18) fermant l'extrémité de distribution ouverte (17) et
d'un dispositif de serrage (S) relié à la tige de piston (35, 135), qui est agencé dans le bloc de réception (10),
le dispositif de serrage (S), lorsque le piston (36) se trouve dans sa position d'extrémité avant, pouvant déplacer la tige de piston (35, 135) dans un processus de serrage le long de la première direction jusqu'à ce que le piston (36) se trouve dans sa position d'extrémité arrière, afin de remplir ainsi le cylindre (16, 116) avec le fluide à appliquer et de précontraindre la tige de piston (35, 135) vers l'extrémité de distribution ouverte (17), et
le dispositif de serrage (S), lorsque le piston (36) se trouve dans sa position d'extrémité arrière, pouvant libérer la tige de piston (35, 135) dans un processus de distribution, de telle sorte que le piston (36), en raison de la précontrainte appliquée, se déplace à l'encontre de la première direction jusqu'à sa position d'extrémité avant et le fluide dans le cylindre (16, 116) est alors distribué pour l'application par l'intermédiaire du clapet antiretour (18),
le cylindre (16, 116) étant configuré conjointement avec le clapet antiretour (18) en tant que groupe frontal échangeable (13, 14), qui est relié de manière amovible au bloc de réception (10),
et le dispositif comprenant une buse (19) pour l'application sans aiguille du fluide, qui est reliée à l'extrémité de distribution ouverte du cylindre (16, 116) par l'intermédiaire du clapet antiretour (18) et qui fait partie du groupe frontal (13, 14).

2. Dispositif selon la revendication 1,
la liaison amovible entre le groupe frontal (13, 14) et le bloc de réception (10) étant une liaison à vis.

3. Dispositif selon l'une quelconque des revendications précédentes,
le dispositif de serrage (S) comprenant un ressort (41, 141), qui précontraint la tige de piston (35, 135) vers l'extrémité de distribution ouverte lorsque le piston (36) se trouve dans la position d'extrémité arrière.

4. Dispositif selon l'une quelconque des revendications précédentes,
le dispositif de serrage (S) comprenant une rampe (52) rotative au moyen d'un moteur (12), munie d'une piste de rampe (53) s'étendant le long d'une hélice,
la piste de rampe (53) montant à partir d'un premier plateau le long d'une zone de montée (S1, S2) jusqu'à un deuxième plateau et descendant à partir du deuxième plateau par l'intermédiaire d'un flanc abrupt (46) jusqu'au premier plateau,
le dispositif de serrage (S) comprenant en outre un rouleau (51) en contact avec la piste de rampe (53), qui est monté de manière rotative dans un entraîneur (50), qui est relié à l'extrémité de la tige de piston (35, 135) dépassant du cylindre (16, 116), de telle sorte que lors d'une rotation de la rampe (52), la piste de rampe (53) passe sous le rouleau (51) qui tourne en conséquence,
pour le processus de serrage, la piste de rampe (53) étant tournée à partir d'un contact du rouleau (51) avec le premier plateau de telle sorte que le rouleau (51) circule sur la zone de montée jusqu'au deuxième plateau et le piston (36) étant ainsi déplacé dans sa position d'extrémité arrière,
pour le processus de distribution, la piste de rampe (53) étant tournée à partir d'un contact du rouleau (51) avec le deuxième plateau jusqu'à ce que le rouleau (51) tombe sur le premier plateau par l'intermédiaire du flanc abrupt (46) et le piston (36) étant ainsi déplacé dans sa position d'extrémité avant.

5. Dispositif selon la revendication 4,
le moteur (12) étant monté dans le bloc de réception (10) .

6. Dispositif selon la revendication 4 ou 5,
la zone de montée de la piste de rampe (53) comprenant une première section (S1) raccordée au premier plateau et une deuxième section (S2) raccordée à celle-ci, la pente de la deuxième section (S2) étant plus grande que la pente de la première section (S1).

7. Dispositif selon la revendication 6,
les deux sections (S1, S2) s'étendant linéairement par rapport à l'angle de rotation de l'hélice.

8. Dispositif selon la revendication 5 ou 6, la longueur d'angle de rotation de la première section (S1) étant inférieure à la longueur d'angle de rotation de la deuxième section (S2).

9. Dispositif selon l'une quelconque des revendications 4 à 8,
le rouleau (51) comprenant une zone d'appui (55), qui s'appuie sur la zone de montée (S1, S2) de la piste de rampe (53), et au moins une zone latérale (56, 57) raccordée latéralement à celle-ci, qui présente un diamètre extérieur plus petit que la zone d'appui (55) et qui ne s'appuie pas sur la zone de montée (S1, S2) de la piste de rampe (53),
lors du processus de distribution, aussi bien la zone d'appui (55) que la zone latérale (56, 57) venant en contact avec un bord (54) de la piste de rampe (53) reliant le deuxième plateau au flanc abrupt (46).

10. Dispositif selon la revendication 9,
le rouleau (51) comprenant sur les deux côtés de la zone d'appui (55) une zone latérale (56, 57) raccordée latéralement, ayant un diamètre extérieur plus petit que celui de la zone d'appui (55).

11. Dispositif selon la revendication 9 ou 10,
le diamètre extérieur de la zone latérale respective (56, 57) diminuant dans une direction allant vers le côté du rouleau (51).

12. Dispositif selon l'une quelconque des revendications 4 à 11,
le rouleau (51) étant monté dans l'entraîneur (50) de telle sorte que son axe de rotation est perpendiculaire à la première direction.

13. Dispositif selon l'une quelconque des revendications précédentes,
le bloc de réception (10) étant configuré sous la forme d'un bloc de réception d'un seul tenant (10), qui est fabriqué par un procédé de fabrication additif.

14. Dispositif selon la revendication 13,
le bloc de réception (10) étant configuré sous la forme d'un bloc de réception métallique (10).

15. Dispositif selon la revendication 13 ou 14,
le bloc de réception (10) comprenant un palier de moteur (64), un cylindre de guidage (40, 140) pour la tige de piston (35, 135), au moins un logement (60, 61, 62, 63) pour une platine de commande (11), un logement (72, 172) pour un raccord de fluide (33) pour un contenant de fluide (M) et/ou au moins un emplacement de fixation de boîtier (65, 66, 67, 68), qui est/sont configuré(s) d'un seul tenant avec le bloc de réception (10) .
